(19) Europäisches Patentamt
European Patent Office
Office européen des brevets

(11) Publication number: 0 195 841 A1

# (12) EUROPEAN PATENT APPLICATION

(21) Application number: 85106626.6

(22) Date of filing: 29.05.85

(51) Int. Cl.⁴: C 07 D 215/56, A 61 K 31/495

(30) Priority: 08.03.85 JP 46216/85

(43) Date of publication of application: 01.10.86 Bulletin 86/40

(84) Designated Contracting States: AT BE CH DE FR GB IT LI LU NL SE

(71) Applicant: KYORIN PHARMACEUTICAL CO., LTD., No. 5, Kanda Surugadai 2-chome, Chiyoda-ku Tokyo (JP)

(72) Inventor: Irakura, Tsutomu, No. 10-28, Ooizumigakuen-cho 7-chome, Nerima-ku Tokyo (JP)
Inventor: Suzue, Seigo, No. 13-4, Aoba 4-chome, Kuki-shi Saitama-ken (JP)
Inventor: Murayama, Satoshi, No. 6095, Tomonuma Nogi-machi, Simotsuga-gun Tochigi-ken (JP)
Inventor: Hirai, Keiji, No. 1-2-512, Aoba 1-chome, Kuki-shi Saitama-ken (JP)
Inventor: Ishizaki, Takayoshi, No. 9-6, Sakurada 4-chome Washimiya-machi, Kitakatsushika-gun Saitama-ken (JP)

(74) Representative: Patentanwälte TER MEER - MÜLLER - STEINMEISTER, Mauerkircherstrasse 45, D-8000 München 80 (DE)

(54) **Quinolinecarboxylic acid derivatives and process for their preparation.**

(57) Quinolinecarboxylic acid derivatives of the following formula,

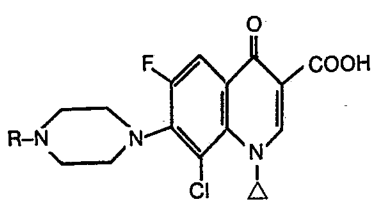

wherein R is hydrogen atom or methyl group; hydrates and pharmaceutically acceptable salts thereof are useful as an antibacterial agent.

EP 0 195 841 A1

Title of the invention:

Quinolinecarboxylic acid derivatives and process for their preparation

Detailed description of the invention:

This invention is concerned with certain novel useful quinolinecarboxylic acid derivatives, with a process for their preparation, and with compositions containing them.

This invention provides compounds of the formula (I),

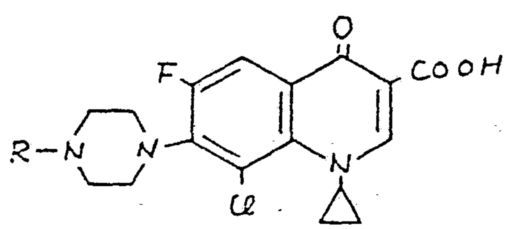

(I)

wherein R is hydrogen atom or methyl group; hydrates and pharmaceutically acceptable salts thereof.

The present inventors had developed Norfloxacin which brought today's prosperity of new quinolone antibacterial agent.

It has been known that quinolones inhibit the DNA gyrase

which controls the topology of DNA.

As the results of our investigation of its mode of action, it was found that antibacterial activity is related to the penetrability of the drug into the bacterial cells through porins in bacterial outer membrane.

Now, after consideration of the above acknowledgement, the present inventors thought the concept on new antibacterial agent as follows:

1) It must inhibit the DNA gyrase.
2) It must have more permeability into bacterial cells through porins.
3) It must be certainly useful for treatment against Gram-positive bacteria, especially Staphylococci.
4) It must have antibacterial activity as well as that of the prior drug against Gram-negative bacteria.
5) It must possess good activity against Serratia sp. and Pseudomonas aeruginosa, which showed resistant to the traditionally used antibiotics.
6) It must be also effective against anaerobic bacteria.
7) It must possess good oral absorbability in human and animals and stability against metabolic inefficiency.
8) It must have lower toxicity and no side effect upon human and animals.
9) It must be excellent in the productivity.

As the compounds satisfied those concepts, the present inventors synthesized the new compounds represented by the formula (I) and investigated their mechanism of action.

The chemical structural feature of the present compounds is that they have fluorine atom on 6-position and chlorine atom on 8-position in quinolone skeleton.

The present inventors previously developed Norfloxacin having fluorine atom at 6-position of quinolone skeleton.

Since Norfloxacin was proved to be clinically very useful for treatment of bacterial infections, quinolonecarboxylic acid analogue has been widely investigated in Japan and abroad. They have brought a remarkable progress to the clinical treatment and are called new quinolones or fluoroquinolones.

As a result of our continuous investigation, we found that substituent in 8-position also played a very important part in extention of antibacterial spectrum and increase in activity and absorbability as a drug.

Then we prepared various compounds having substituent at 8-position and tested their antibacterial activity. The results were analized by means of Quantitative Structure Activity Relationship and so on. It was found unexpectedly that chlorine atom is the best as substituent at 8-position.

The present compound having chlorine atom at 8-position shows stronger activity against, for example, Gram-positive and anaerobic bacteria than the analogue having hydrogen or fluorine atom at 8-position.

Furthermore, the present compound shows comparable activity against Gram-negative bacteria and greater activity against Serratia marcescens and Pseudomonas aeruginosa, which showed resistant to various antibiotics, in comparison with

Ciprofloxacin which possesses the strongest activity against Gram-negative bacteria among the compound of the prior art.

On the other hand, the present compounds show good stability in a living body and low toxicity in animals.

The compounds of the formula (I) are synthesized by reacting a compound of the formula (II),

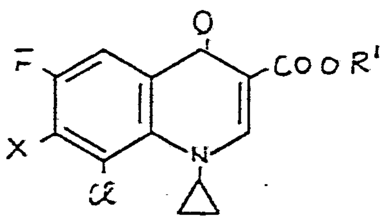

(II)

wherein X is halogen atom and $R^1$ is hydrogen atom or lower alkyl group, with a compound of the formula (III),

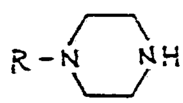

(III)

wherein R is hydrogen atom or methyl group. The reaction is preferably carried out by heating the two reactants in a solvent such as water, alcohols, acetonitrile, dimethylformamide, dimethyl sulfoxide, hexamethylphosphoric triamide, pyridine, picoline and the like or in absence of the solvent with, if necessary, an acid acceptor such as an inorganic or organic acceptor, e.g., alkali metal carbonate or *tert* -amine, at a temperature of room temperature to 200°C, preferably room temperature to 160°C. It is desirable that a slight excess (1 to 5 moles) of the secondary amine of the formula (III) is used per mole of the formula (II) and a solvent is used such that the mixture remains homogeneous after dissolution of the compound of the formula (II) (2 to 10-fold volume of the compound of the

formula (III).

When $R^1$ in the formula (II) is lower alkyl group, the reaction product (carboxylic ester) is saponified to the corresponding carboxylic acid by the usual manner.

The saponification is carried out by treating the compound of formula (IV) with alkali metal hydroxide solution such as sodium hydroxide, potassium hydroxide, or mineral acid such as hydrochloric acid, sulfuric acid in water, aqueous alcohols or acetic acid containing water at a room temperature to boiling point of the used solvent.

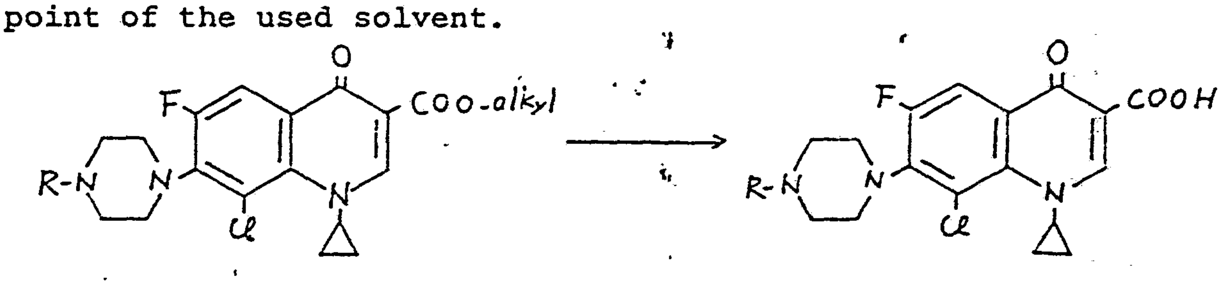

(IV) (I)

The starting compounds of the formula (II) are also new and obtained according to the following route.

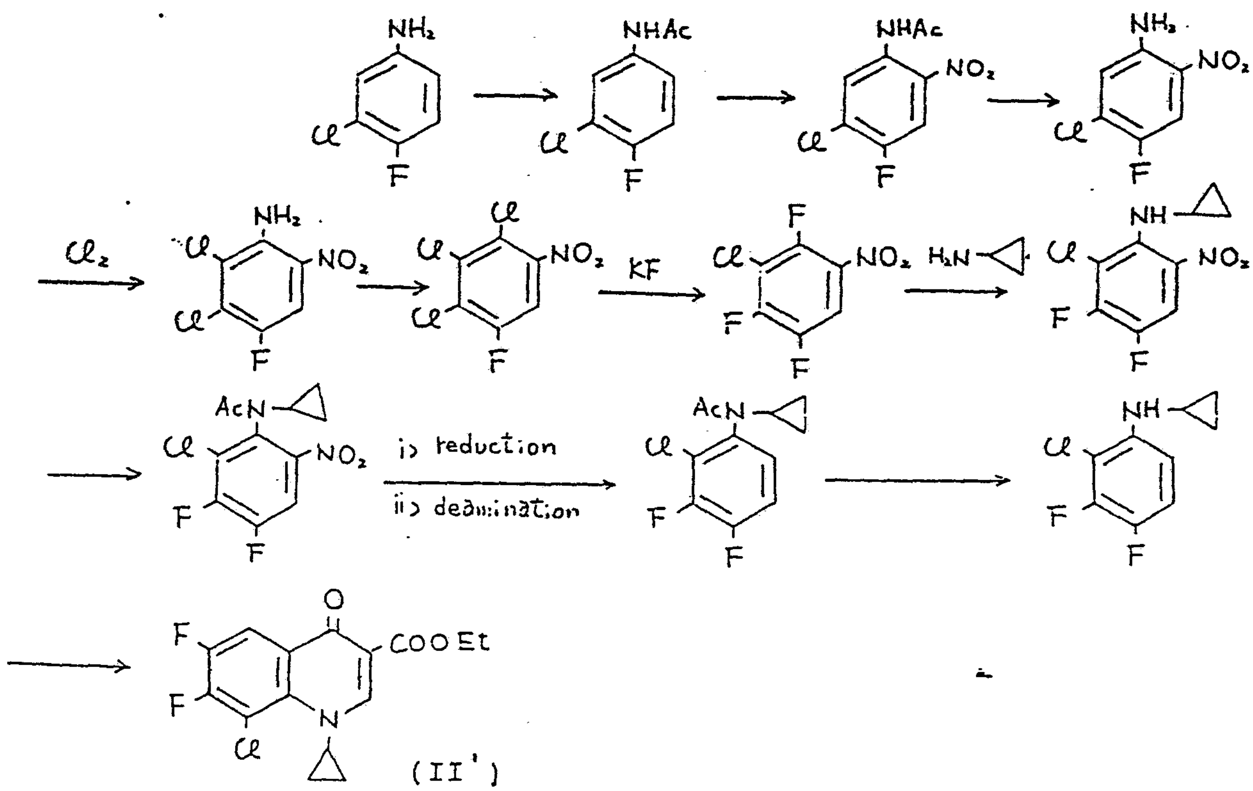


(II')

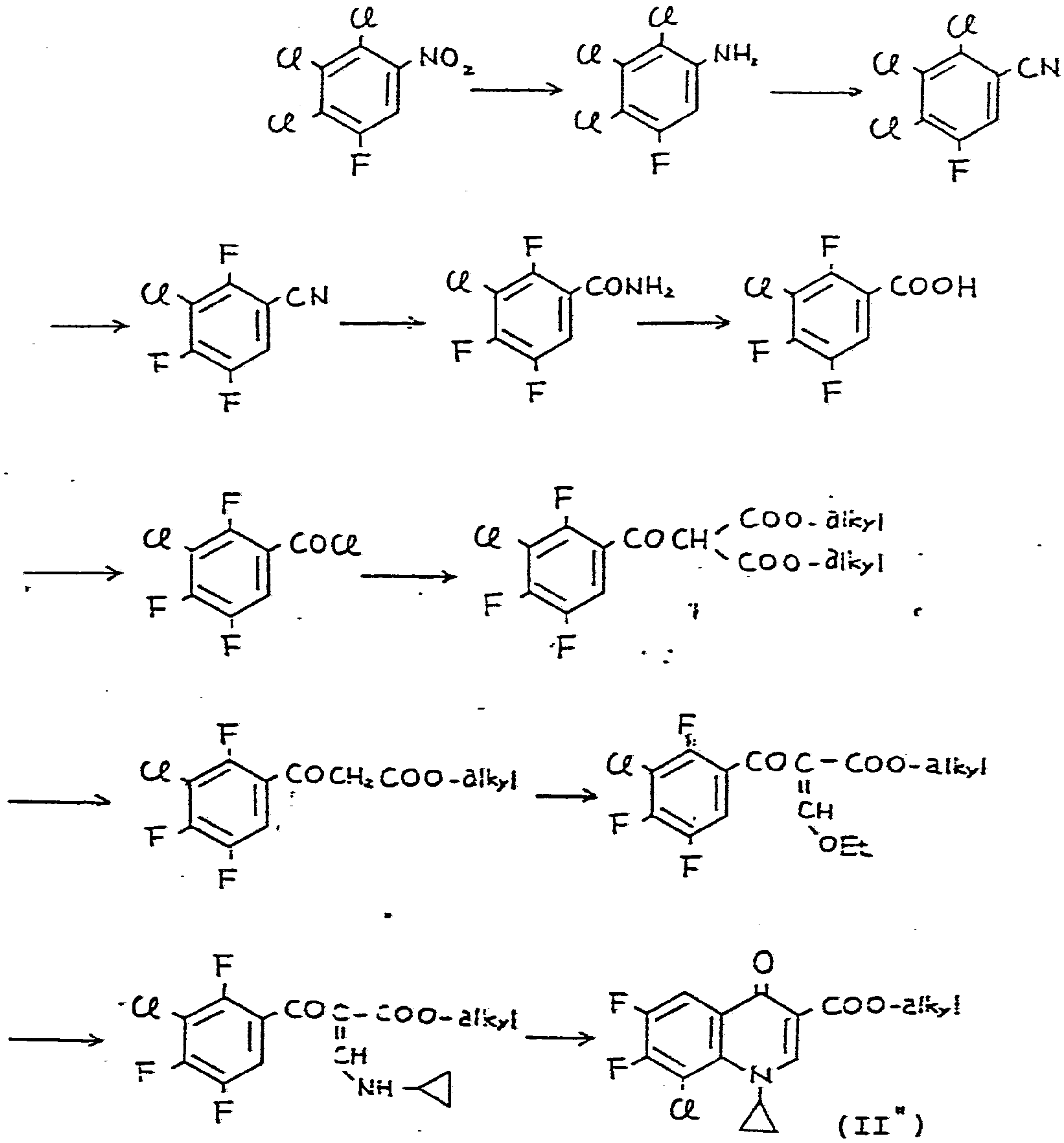

(II")

Furthermore, the compounds of the formula (I) can be converted, if desired, to the pharmaceutically acceptable ammonium salts or carboxylic acid metal salts by treatment with acid or alkali. The acid may be organic or inorganic acids such as, for example, hydrochloric acid, sulfuric acid, phosphoric acid, acetic acid, methanesulfonic acid, oxalic acid and lactic acid. The carboxylic acid metal salts may be, for example, sodium, potassium, magnesium, calcium, aluminum, cerium, chromium, cobalt, copper, iron, zinc, platinum and silver salts.

The compounds of the formula (I), hydrates and salts thereof may be used as medicines in the conventional form of pharmaceutical preparations, which may be, for example, tablets, capsules, powder, ointments, suppositories, injections or eye drops, suitable for peroral, parenteral, enteral or local administration.

The following examples will further illustrate the invention without, however, limiting it thereto.

Example 1 N-(3-chloro-4-fluorophenyl)acetamide

To 3-chloro-4-fluoroaniline (100 g) was slowly added acetic anhydride (200 ml). After allowed to stand for 30 minutes, the reaction mixture was poured into water (1 litter).
The resulting precipitate was collected by filtration and recrystallized from aqueous ethanol to give the title compound (119.4 g), mp 118-119°C.

Example 2 N-(3-chloro-4-fluoro-6-nitrophenyl)acetamide

To a solution of N-(3-chloro-4-fluorophenyl)acetamide (55 g) in concentrated sulfuric acid (165 ml) was added dropwise

concentrated nitric acid (d 1.42, 154 ml) at 5-10°C during an hour with stirring in an ice-salt bath. After stirring for an hour at the same temperature, the reaction mixture was poured into ice water. The resulting precipitate was collected by filtration, sufficiently washed with water and recrystallized from acetonitrile to give the title compound (48.9 g) as yellow needles, mp 114-115°C.

Analysis (%) for $C_8H_6ClFN_2O_3$, Calcd. (Found): C, 41.31 (41.48); H, 2.60 (2.52); N, 12.04 (12.13).

Example 3 3-Chloro-4-fluoro-6-nitroaniline

A solution of N-(3-chloro-4-fluoro-6-nitrophenyl)acetamide (30 g) in concentrated hydrochloric acid (50 ml) and ethanol (200 ml) was refluxed for 2.5 hours. To the reaction mixture was added ice water (300 ml) and the resulting precipitate was collected by filtration, washed with water and dried to give the title compound (24.9 g) as yellow needles, mp 149.5-150°C.

Analysis (%) for $C_6H_4ClFN_2O_2$, Calcd. (Found): C, 37.82 (37.85); H, 2.11 (2.03); N, 14.70 (14.80).

Example 4 2,3-Dichloro-4-fluoro-6-nitroaniline

Into a solution of 3-chloro-4-fluoro-6-nitroaniline (14.3 g) in acetic acid (150 ml) was boubled chlorine gas at 18-20°C during 70 minutes. The reaction mixture was poured into ice water (300 ml) and the resulting precipitate was collected by filtration, washed with water and recrystallized from ethanol to give the title compound (14.33 g) as yellow needles, mp 161°C.

Analysis (%) for $C_6H_3Cl_2FN_2O_2$, Calcd. (Found) :C, 32.03 (32.17); H, 1.34 (1.26); N, 12.45 (12.65).

Example 5 2,3,4-Trichloro-5-fluoronitrobenzene

To a mixture of anhydrous cupric chloride (13.58 g) and *tert*-butylnitrite (12.4 g) in anhydrous acetonitrile (100 ml) was added portionwise 2,3-dichloro-4-fluoro-6-nitroaniline (18.05 g) at 60-62°C during 30 minutes. After stirring for 30 minutes at 60-65°C, the reaction mixture was poured into chilled 10% diluted hydrochloric acid (300 ml) and extracted with benzene.
The organic layer was successively washed with diluted hydrochloric acid and water, dried over anhydrous sodium sulfate and concentrated. The resulting residue was purified by distillation to give the title compound(17.26 g), bp 137-142°C/27 mmHg.

NMR ( δ in $CDCl_3$), 7.65 (d, J=7.5 Hz)

Example 6 3-Chloro-2,4,5-trifluoronitrobenzene

To a suspension of potassium fluoride (64.9 g) in anhydrous dimethyl sulfoxide (230 ml) was added 2,3,4-trichloro-5-fluoronitrobenzene (54.4 g) at 140°C and stirred at the same temperature for 10 minutes. The reaction mixture was poured into ice water (700 ml) and extracted with petroleum ether.
The organic layer was successively washed with water, aqueous potassium carbonate solution and then water, dried over anhydrous sodium sulfate and concentrated. The resulting residue was distilled to give the title compound (9.7 g), bp 95-108°C/30mmHg.

NMR ( δ in $CDCl_3$), 7.94 (ddd, J=6.7, 7.6, 9.0 Hz)

Example 7 3-Chloro-2-cyclopropylamino-4,5-difluoronitrobenzene

A solution of cyclopropylamine (2.8 g) and triethylamine (5.1 g) in anhydrous toluene (20 ml) was added dropwise to a

solution of 3-chloro-2,4,5-trifluoronitrobenzene (9.7 g) in anhydrous toluene (30 ml) at 3-5°C during 40 minutes with stirring. After stirring for 3 hours at the same temperature, the reaction mixture was poured into ice water (150 ml) and extracted with dichloromethane. The organic layer was washed with water, dried over anhydrous sodium sulfate and then concentrated. The resulting residue was purified by silica gel chromatography using n-hexane-dichloromethane as an eluent to give the title compound (4.4 g) as red oil.

NMR ( δ in $CDCl_3$), 0.5-1.0 (4H, m, 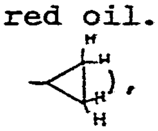), 3.0-3.2 (1H, m, 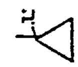), 7.19 (1H, s, NH), 7.85 (1H, dd, J=8.2, 9.9 Hz, 5-H).

Example 8 N-(2-Chloro-3,4-difluoro-6-nitrophenyl)-N-cyclopropylacetamide

To 3-chloro-2-cyclopropylamino-4,5-difluoronitrobenzene (4.4 g) was added acetic anhydride (15 ml) with one portion and the mixture was stirred for 30 minutes at room temperature, and then poured into ice water (100 ml). Excessive acetic anhydride was decomposed by potassium carbonate powder, and then the mixture was allowed to stand overnight at 5°C.

The resulting precipitate was collected by filtration and recrystallized from ethyl acetate-n-hexane to give the title compound (2.7 g), mp 98-99.5°C.

Analysis (%) for $C_{11}H_9ClF_2N_2O_3$, Calcd. (Found): C, 45.46 (45.56); H, 3.12 (3.00); N, 9.64 (9.69).

Example 9 N-(2-Chloro-3,4-difluorophenyl)-N-cyclopropylacetamide

A mixture of N-(2-chloro-3,4-difluoro-6-nitrophenyl)-N-cyclopropylacetamide (2.7 g) and 10% palladium-charcoal (0.5 g) in ethanol (50 ml) was hydrogenated for 40 minutes at 2-3°C under atmospheric pressure in an ice bath. The catalyst was filtered off and the filtrate was concentrated. Then, the resulting crystalline residue was dried in vacuo at room temperature for 10 hours. A solution of the residue in anhydrous dimethylformamide (15 ml) was added dropwise to a solution of *tert*-butyl nitrite (1.72 g) in anhydrous dimethylformamide (10 ml) at 50-52°C during 13 minutes. The reaction mixture was stirred at the same temperature for 5 minutes, then poured into ice water and extracted with ether. The organic layer was successively washed with water, dilute hydrochloric acid and water, dried over anhydrous sodium sulfate and concentrated. The resulting residue was purified by silica gel chromatography using n-hexane-ethyl acetate as an eluent and recrystallized from petroleum ether to give the title compound (0.44 g), mp 60.5-61.5°C.

Analysis (%) for $C_{11}H_{10}ClF_2NO$, Calcd. (Found): C, 53.78 (53.87); H, 4.10 (4.02); N, 5.70 (5.78).

Example 10 N-Cyclopropyl-2-chloro-3,4-difluoroaniline

A solution of N-(2-chloro-3,4-difluorophenyl)-N-cyclopropylacetamide (0.44 g) in 20% diluted hydrochloric acid (7 ml) was heated at 80-100°C for 6 hours with stirring, and then cooled. The reaction mixture was poured into ice water, alkalized with aqueous sodium hydroxide and extracted with ether. The ether layer was washed with water, dried over anhydrous sodium sulfate and then concentrated. The resulting residue was purified by silica gel chromatography using petroleum ether as an eluent to

give the title compound (100 mg) as orange oil.

Example 11 Ethyl 8-chloro-1-cyclopropyl-6,7-difluoro-1,4-dihydro-4-oxoquinoline-3-carboxylate

A mixture of N-cyclopropyl-2-chloro-3,4-difluoroaniline (100 mg) and diethyl ethoxymethylenemalonate (100 mg) was stirred for 10.5 hours at 100-135°C with removal of generated ethanol by flowing nitrogen gas, and then cooled. Polyphosphoric acid (1 g) was mixed with this, and the mixture was stirred for 3 hours at 125-135°C. After cooling, the reaction mixture poured into ice water, extracted with chloroform. The organic layer was successively washed with aqueous potassium carbonate and water, dried over anhydrous sodium sulfate and then concentrated. The resulting residue was purified with silica gel thin layer chromatography using ether as an eluent to give the title compound (11 mg) as colorless needles, mp 160-162.5°C.

NMR ( δ in $CDCl_3$), 1.0-1.5 (4H, m, 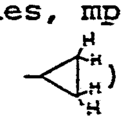), 1.40 (3H, t, J=7.0 Hz, $-\underline{CH_3}$), 4.1-4.4 (1H, m, 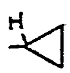 ), 4.38 (2H, q, J=7.0 Hz, $-\underline{CH_2}-CH_3$), 8.22 (1H, dd, J=8.8, 9.7 Hz, 5-H), 8.66 (1H, s, 2-H)

Example 12 2,3,4-Trichloro-5-fluoroaniline

To a suspension of iron powder (54.6 g) in water (60 ml), with vigorous stirring at 50-60°C, was slowly added concentrated hydrochloric acid (6.7 ml). After ethanol (150 ml) was mixed, 2,3,4-trichloro-5-fluoronitrobenzene (75.1 g) was added portionwise to the suspension at 60-70°C during an hour. After stirring for an hour at 80°C, the hot reaction mixture was filtered and the insoluble material was successively washed with hot ethanol (100 ml) and benzene (300 ml). The filtrate and

washings were combined and mixed with ice water. The resulting organic layer was collected and the water layer was extracted with benzene (200 ml). The organic layers were washed with water, dried over anhydrous sodium sulfate and then concentrated. The resulting residue was recrystallized from n-hexane to give the title compound (58.6 g) as light brown needles, mp 118-120°C.

Example 13 2,3,4-Trichloro-5-fluorobenzonitrile

To a suspension of 2,3,4-trichloro-5-fluoroaniline (43.8 g) in concentrated hydrochloric acid (300 ml) with vigorous stirring was added sodium nitrite (21.1 g) in water (50 ml) at -2 ∿ 0°C for 20 minutes. After stirred for 30 minutes, the mixture was poured into ice water (300 ml) containing sodium tetrafluoroborate (67.2 g), stirred vigorously and then allowed to stand for 30 minutes in an ice bath. The resulting precipitate was collected by filtration and successively washed with chilled water and ether. This faint yellow precipitate was added portionwise during 30 minutes to a solution of cuprous cyanide (36.5 g), potassium cyanide (53.0 g) and sodium carbonate (11.1 g) in water (300 ml) with vigorous stirring at room temperature. After the mixture was stirred for 30 minutes, benzene (300 ml) was added to the suspension and then the mixture was stirred for 15 minutes. The insoluble material was collected by filtration, and washed with benzene (150 ml). The filtrate and washings were combined and successively washed with 20% aqueous potassium cyanide and water, dried over anhydrous sodium sulfate and then concentrated. The resulting residue was recrystallized from n-hexane to give the title compound (27 g) as light brown needles, mp 97-99°C.

Example 14 3-Chloro-2,4,5-trifluorobenzonitrile

To a solution of potassium fluoride (31.7 g) in dimethyl sulfoxide (100 ml) with stirring at 130°C was added 2,3,4-trichloro-5-fluorobenzonitrile (15 g) and then the mixture was stirred for 1.5 hours at 140°C. After cooling, the reaction mixture was poured into ice water (300 ml) and extracted with dichloromethane. The organic layer was washed with water, dried over anhydrous sodium sulfate and concentrated to give the title compound (11.9 g) as pale brown oil.

Example 15 3-Chloro-2,4,5-trifluorobenzamide

A solution of 3-chloro-2,4,5-trifluorobenzonitrile (11.9 g) in 30% hydrogen bromide-acetic acid (150 ml) was refluxed for 80 minutes, poured into ice water (350 ml) and extracted with ether. The ether layer was successively washed with 1N-potassium hydroxide solution and water, dried over anhydrous sodium sulfate and concentrated. The residue was purified by silica gel chromatography eluting with n-hexane-ethyl acetate to give the title compound (3.97 g), mp 110-113.5°C.

Example 16 3-Chloro-2,4,5-trifluorobenzoic acid

A mixture of 3-chloro-2,4,5-trifluorobenzamide (3.97 g) and 18N-sulfuric acid (20 ml) was stirred at 125-135°C for 9 hours, and then poured into ice water (100 ml). After standing overnight, the resulting precipitate was collected by filtration. The mother liquor was extracted with ether, and the ether layer was dried over anhydrous sodium sulfate, then concentrated and mixed to the precipitate. A solution of the above precipitate and residue in dichloromethane (150 ml) was filtered through a

celite pad and the filtrate was concentrated to give the title compound (2.38 g), mp 115-116.5°C.

Analysis (%) for $C_7H_2ClF_3O_2$, Calcd. (Found): C, 39.93 (40.18); H, 0.96 (0.80).

Example 17 3-Chloro-2,4,5-trifluorobenzoyl chloride

A solution of the 3-chloro-2,4,5-trifluorobenzoic acid (2.38 g) in thionyl chloride (10 ml) was refluxed for 2.5 hours, and then concentrated. The resulting residue was purified by distillation in nitrogen atomosphere to give the title compound (1.99 g), bp 88°C/19 mmHg.

Example 18 Diethyl 3-chloro-2,4,5-trifluorobenzoylmalonate

Magnesium turnings (0.22 g) and carbon tetrachloride (0.1 ml) was added to absolute ethanol (1.5 ml). To the stirring suspension was added dropwise a solution of diethyl malonate (1.4 g) and absolute ethanol (2 ml) in toluene (6 ml) during 28 minutes at 47-60°C. The mixture was stirred for 80 minutes, and then cooled in an acetone-dry ice bath. A solution of 3-chloro-2,4,5-trifluorobenzoyl chloride (1.99 g) in anhydrous toluene (2 ml) was added dropwise to the resulting solution at -12 ∿-8°C during 13 minutes. The mixture was stiired for 2 hours at -10∿ -5°C allowed to stand overnight at room temperature, and then mixed with ice water (6 ml) containing concentrated sulfuric acid (0.4 ml). The resulting organic layer was collected and the water layer was extracted with toluene. The combined organic layer was washed with water, dried over anhydrous sodium sulfate and then concentrated to give the title compound (3.05 g) as pale yellow oil.

Example 19 Ethyl 3-chloro-2,4,5-trifluorobenzoylacetate

To an emulsion of diethyl 3-chloro-2,4,5-trifluorobenzoylmalonate (3.05 g) in water (4 ml) was added p-toluenesulfonic acid (4 mg) and refluxed for 4 hours with vigorous stirring. After cooling, the reaction mixture was extracted with dichloromethane. The organic layer was washed with water, dried over anhydrous sodium sulfate and concentrated. The residue was recrystallized from ether-n-hexane to give the title compound (1.22 g), mp 80-83°C.

Analysis (%) for $C_{11}H_8ClF_3O_3$, Calcd. (Found): C, 47.08 (46.96); H, 2.87 (2.77).

Example 20 Ethyl 2-(3-chloro-2,4,5-trifluorobenzoyl)-3-ethoxyacrylate

A mixture of ethyl 3-chloro-2,4,5-trifluorobenzoylacetate (1.22 g), ethyl orthoformate (0.97 g) and acetic anhydride (1.12 g) was stirred at 118-143°C for 3 hours and then concentrated to give the title compound (1.4 g) as yellow oil.

Example 21 Ethyl 2-(3-chloro-2,4,5-trifluorobenzoyl)-3-cyclopropylaminoacrylate

To a solution of ethyl 2-(3-chloro-2,4,5-trifluorobenzoyl)-3-ethoxyacrylate (1.4 g) in absolute ethanol (3 ml) was added a solution of cyclopropylamine (0.26 g) in absolute ethanol (2 ml) at 5-10°C during 15 minutes. The mixture was allowed to stand at 5°C for 1.5 hours and then stirred at room temperature for an hour. The resulting precipitate was collected by filtration. The filtrate was concentrated, mixed with the precipitate and then recrystallized from petroleum ether to give the title

compound (1.09 g), mp 84-85.5°C.

Analysis (%) for $C_{15}H_{13}ClF_3NO_3$, Calcd. (Found): C, 51.81 (51.76); H, 3.77 (3.74); N, 4.03 (4.03).

Example 22 Ethyl 8-chloro-1-cyclopropyl-6,7-difluoro-1,4-dihydro-4-oxo-quinoline-3-carboxylate

To a solution of ethyl 2-(3-chloro-2,4,5-trifluorobenzoyl)-3-cyclopropylaminoacrylate (1.09 g) in anhydrous dimethylforamide (5 ml) was added sodium fluoride (0.21 g). The mixture was stirred at 130-156°C for 3.5 hours, and then poured into ice water (50 ml) and the resulting precipitate was collcted by filtration, washed with water and recrystallized from ethyl acetate to give the title compound (0.96 g), mp 158-159°C.

Analysis (%) for $C_{15}H_{12}ClF_2NO_3$, Calcd. (Found): C, 54.98 (54.96); H, 3.69 (3.57); N, 4.27 (4.25).

Example 23 8-Chloro-1-cyclopropyl-6,7-difluoro-1,4-dihydro-4-oxoquinoline-3-carboxylic acid

A mixture of ethyl 8-chloro-1-cyclopropyl-6,7-difluoro-1,4-dihydro-4-oxoquinoline-3-carboxylate (0.24 g), acetic acid (2 ml), water (1.5 ml) and concentrated sulfuric acid (0.25 ml) was refluxed for an hour, and then poured into ice water. The resulting precipitate was collected by filtration and successively washed with water and ether to give the title compound (0.17 g), mp 194-195°C.

Analysis (%) for $C_{13}H_8ClF_2NO_3$, Calcd. (Found): C, 52.11 (52.00); H, 2.69 (2.53); N, 4.67 (4.64).

Example 24 Ethyl 8-chloro-1-cyclopropyl-6-fluoro-1,4-dihydro-4-oxo-7-(1-piperazinyl)quinoline-3-carboxylate

To a solution of anhydrous piperazine (0.39 g) in dimethyl sulfoxide was added ethyl 8-chloro-1-cyclopropyl-6,7-difluoro-1,4-dihydro-4-oxoquinoline-3-carboxylate (0.30 g), and then stirred at 50-60°C for 75 minutes. After cooling, the mixture was mixed with chloroform (10 ml) and aqueous potassium carbonate solution. The resulting organic layer was collected and the water layer was extracted with chloroform (5 ml) 3 times. The combined organic layer was extracted with 3% hydrochloric acid (5 ml) 3 times. The water layer was ajusted to pH 10-11 with aqueous sodium hydroxide and quickly extracted with chloroform. The chloroform layer was washed with water, dried over anhydrous sodium sulfate and concentrated. The residue was triturated with acetone (10 ml) and the resulting precipitate was collected by filtration to give the title compound (0.08 g) as colorless needles, mp 184-186°C.

Analysis (%) for $C_{19}H_{21}ClFN_3O_3$, Calcd. (Found): C, 57.94 (57.85); H, 5.37 (5.20); N, 10.67 (10.58).

Example 25 8-Chloro-1-cyclopropyl-6-fluoro-1,4-dihydro-4-oxo-7-(1-piperazinyl)quinoline-3-carboxylic acid and its hydrochloride

A suspension of ethyl 8-chloro-1-cyclopropyl-6-fluoro-1,4-dihydro-4-oxo-7-(1-piperazinyl)quinoline-3-carboxylate (70 mg) in 1N sodium hydroxide solution (1.5 ml) was stirred at 80-85°C for 30 minutes. The resulting hot solution was treated with charcoal and filtered. The filtrate was neutralized with acetic acid and then allowed to stand overnight. The resulting precipitate was collected by filtration to give the title compound (42 mg) as

colorless prisms, mp 244.5-246.5°C (decompd.).

Analysis (%) for $C_{17}H_{17}ClFN_3O_3 \cdot H_2O$, Calcd. (Found): C, 53.20 (53.29); H, 4.99 (4.70); N, 10.95 (10.88).

After addition of concentrated hydrochloric acid (5 drops) to the above mother liquor, the resulting precipitate was collected by filtration and successively washed with chilled water and ether to give its hydrochloride (13 mg) as colorless prisms, mp 260°C (decompd.).

Analysis (%) for $C_{17}H_{17}ClFN_3O_3 \cdot HCl$, Calcd. (Found): C, 50.76 (50.66); H, 4.51 (4.55); N, 10.45 (10.37).

Example 26 Ethyl 8-chloro-1-cyclopropyl-6-fluoro-1,4-dihydro-7-(4-methyl-1-piperazinyl)-4-oxoquinoline-3-carboxylate

Reaction of ethyl 8-chloro-1-cyclopropyl-6,7-difluoro-1,4-dihydro-4-oxoquinoline-3-carboxylate (0.30 g) with N-methylpiperazine (0.19 g) in a similar manner as Example 24 gave the title compound (0.12 g) as light brown needles, after recrystallization of the crude product from ethyl acetate, mp 197-198°C.

Analysis (%) for $C_{20}H_{23}ClFN_3O_3$, Calcd. (Found): C, 58.90 (58.84); H, 5.68 (5.67); N, 10.30 (10.24).

Example 27 8-Chloro-1-cyclopropyl-6-fluoro-1,4-dihydro-7-(4-methyl-1-piperazinyl)4-oxoquinoline-3-carboxylic acid and its hydrochloride

Saponification of ethyl 8-chloro-1-cyclopropyl-6-fluoro-1,4-dihydro-7-(4-methyl-1-piperazinyl)-4-oxoquinoline-3-carboxylate (110 mg) in a similar manner as Example 25 gave the title compound (2 mg) as light brown needles, after collection by

filtration from the neutralized reaction mixture.

MS. (m/e): 381 ($M^+$+2), 379 ($M^+$), 335, 299.

And then, addition of several drops of concentrated hydrochloric acid to the above mother liquor gave its hydrochloride (79 mg) as colorless needles, mp 218°C (decompd.).

Analysis (%) for $C_{18}H_{19}ClFN_3O_3 \cdot HCl \cdot 4/5\ H_2O$: C, 50.20 (50.13); H, 5.05 (4.75); N, 9.76 (9.79).

Experiment 1. Antibacterial spectra

Antibacterial activity was determined with the standard method recommended by Japan Society of Chemotherapy. The results were shown in Table 1. Bracketed numbers indicate the ratio of activity of the each present compound per that of Ciprofloxacin.

The present compound, Example 25 and 27, showed much higher antibacterial activity against obligate anaerobic and aerobic Gram-positive bacteria than corresponding compounds that have hydrogen (Ciprofloxacin) or fluorine (reference compound 1 and 2) atom at 8-position of quinolone skeleton.

The present compounds also showed excellent activity against Gram-negative bacteria including Serratia marcescens and Pseudomonas aeruginose.

Table - 1

| Organism ( $10^6$ cells/ml ) | Gram | Minimum Inhibitory Concentration ( μg/ml ) | | | | |
|---|---|---|---|---|---|---|
| | | CFLX*1 | Reference - 1 *2 | Example 25 | Reference - 2 *3 | Example 27 |
| Bacillus subtilis PCI 219 | + | 0.05 ( 1 ) | 0.025 ( 2 ) | 0.0125 ( 4 ) | 0.05 ( 1 ) | 0.0125 ( 4 ) |
| Staphylococcus aureus 209 P | + | 0.39 ( 1 ) | 0.20 ( 2 ) | 0.05 ( 8 ) | 0.10 ( 4 ) | 0.10 ( 4 ) |
| S. aureus IID 670 ( Terajima ) | + | 0.39 ( 1 ) | 0.20 ( 2 ) | 0.10 ( 4 ) | 0.20 ( 2 ) | 0.10 ( 4 ) |
| S. epidermidis IID 866 | + | 0.39 ( 1 ) | 0.20 ( 2 ) | 0.05 ( 8 ) | 0.20 ( 2 ) | 0.10 ( 4 ) |
| Streptococcus pyogenes IID 692 | + | 0.78 ( 1 ) | 0.78 ( 1 ) | 0.39 ( 2 ) | 0.78 ( 1 ) | 0.20 ( 4 ) |
| S. pyogenes IID 689 ( S - 8 ) | + | 0.39 ( 1 ) | 0.39 ( 1 ) | 0.20 ( 2 ) | 0.78 ( 0.5 ) | 0.20 ( 2 ) |
| S. faecalis IID 682 | + | 0.78 ( 1 ) | 0.78 ( 1 ) | 0.20 ( 4 ) | 0.78 ( 1 ) | 0.39 ( 2 ) |
| S. pneumoniae 2054 | + | 1.56 ( 1 ) | 0.78 ( 2 ) | 0.39 ( 4 ) | 0.78 ( 2 ) | 0.39 ( 4 ) |
| E. coli NIHJ JC - 2 | − | ≤ 0.006 ( 1 ) | ≤ 0.006 ( 1 ) | ≤ 0.006 ( 1 ) | 0.0125 ( 0.5 ) | ≤ 0.006 ( 1 ) |
| E. coli ATCC 10536 | − | 0.0125 ( 1 ) | 0.0125 ( 1 ) | ≤ 0.006 ( 2 ) | 0.0125 ( 1 ) | 0.0125 ( 1 ) |
| Proteus vulgaris IFO 3167 | − | ≤ 0.006 ( 1 ) | ≤ 0.006 ( 1 ) | ≤ 0.006 ( 1 ) | 0.025 ( 0.25 ) | 0.0125 ( 0.5 ) |
| P. mirabilis IID 994 | − | 0.0125 ( 1 ) | ≤ 0.006 ( 2 ) | 0.0125 ( 1 ) | 0.025 ( 0.5 ) | 0.025 ( 0.5 ) |
| P. morganii IID 602 | − | 0.05 ( 1 ) | 0.025 ( 2 ) | 0.0125 ( 4 ) | 0.05 ( 1 ) | 0.05 ( 1 ) |
| Klebsiella pneumoniae KY 6445 | − | 0.0125 ( 1 ) | 0.0125 ( 1 ) | 0.0125 ( 1 ) | 0.0125 ( 1 ) | 0.025 ( 0.5 ) |
| Enterobacter cloacae IID 977 | − | 0.05 ( 1 ) | 0.025 ( 2 ) | 0.025 ( 2 ) | 0.05 ( 1 ) | 0.05 ( 1 ) |
| Citrobacter freundii IID 976 | − | 0.006 ( 1 ) | 0.0125 ( 0.5 ) | 0.0125 ( 0.5 ) | 0.025 ( 0.25 ) | 0.025 ( 0.25 ) |
| Serratia marcescens IID 618 | − | 0.39 ( 1 ) | 0.10 ( 4 ) | 0.025 ( 16 ) | 0.39 ( 1 ) | 0.05 ( 8 ) |
| Shigella sonnei IID 969 | − | ≤ 0.006 ( 1 ) | ≤ 0.006 ( 1 ) | ≤ 0.006 ( 1 ) | 0.0125 ( 0.5 ) | 0.0125 ( 0.5 ) |
| Salmonella enteritidis IID 604 | − | 0.025 ( 1 ) | 0.0125 ( 2 ) | 0.0125 ( 2 ) | 0.05 ( 0.5 ) | 0.05 ( 0.5 ) |
| Pseudomonas aeruginosa V - 1 | − | 0.20 ( 1 ) | 0.20 ( 1 ) | 0.05 ( 4 ) | 0.39 ( 0.5 ) | 0.20 ( 1 ) |
| Pseudomonas aeruginosa IFO 12689 | − | 0.39 ( 1 ) | 0.39 ( 1 ) | 0.39 ( 1 ) | 0.39 ( 1 ) | 1.56 ( 0.25 ) |
| Yersinia enterocolitica IID 981 | − | 0.05 ( 1 ) | 0.025 ( 2 ) | 0.025 ( 2 ) | 0.05 ( 1 ) | 0.05 ( 1 ) |
| Acinetobacter anitratus IID 876 | − | 0.025 ( 1 ) | 0.0125 ( 2 ) | 0.05 ( 0.5 ) | 0.0125 ( 2 ) | 0.025 ( 1 ) |
| Alcaligenes faecalis 0104002 | − | 0.39 ( 1 ) | 0.020 ( 2 ) | 0.020 ( 2 ) | 0.020 ( 2 ) | 0.39 ( [illegible] ) |
| Bacteroides fragilis GM 7000 | − | 3.13 ( 1 ) | 1.56 ( 2 ) | 0.39 ( 8 ) | 0.78 ( 4 ) | 0.20 ( 16 ) |
| Bacteroides fragilis 0558 | − | 3.13 ( 1 ) | 0.78 ( 4 ) | 0.20 ( 16 ) | 0.39 ( 8 ) | 0.10 ( 32 ) |
| Bacteroides fragilis 25285 | − | 3.13 ( 1 ) | 0.78 ( 4 ) | 0.20 ( 16 ) | 0.39 ( 8 ) | 0.10 ( 32 ) |

CFLX : 1-Cyclopropyl-6-fluoro-1,4-dihydro-4-oxo-7-(1-piperazinyl)quinoline-3-carboxylic acid ( Ciprofloxacin )

Reference - 1 : 1-Cyclopropyl-6,8-difluoro-1,4-dihydro-4-oxo-7-(1-piperazinyl)quinoline-3-carboxylic acid

Reference - 2 : 1-Cyclopropyl-6,8-difluoro-1,4-dihydro-7-(4-methyl-1-piperazinyl)-4-oxoquinoline-3-carboxylic acid

*1

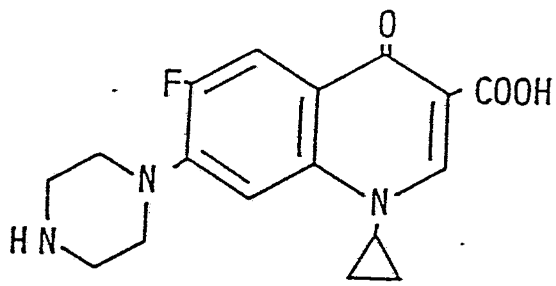

*2

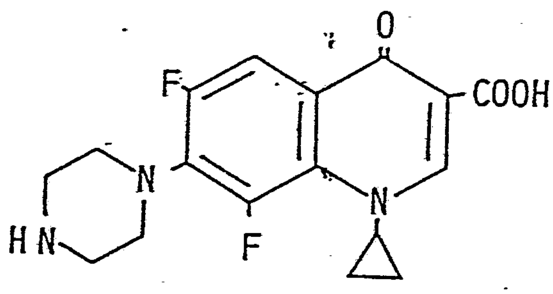

*3

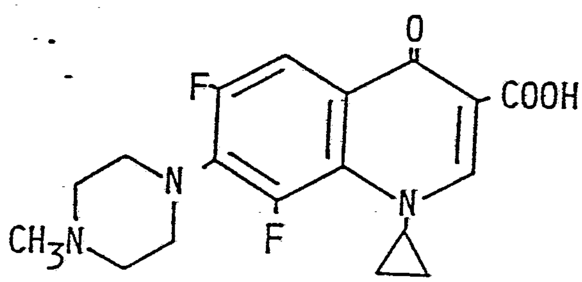

Claims:

1. A compound of the formula (I);

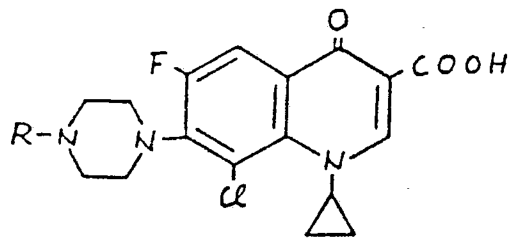

(I)

wherein R is a hydrogen atom or a methyl group; the hydrates or the pharmaceutical acceptable acid addition or alkali salts thereof.

2. A process for the preparation of a compound of the formula (I)

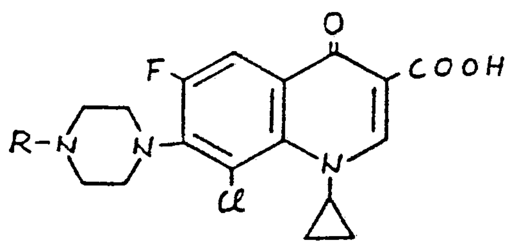

(I)

wherein R is a hydrogen atom or a methyl group; the hydrates or the paramaceutical acceptable acid addition or alkali salts thereof

c h a r a c t e r i z e d b y condensing a compound of the formula (II);

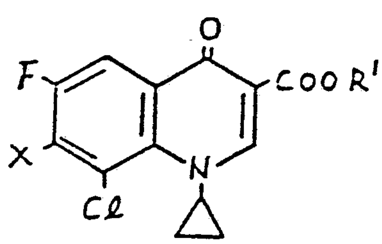

(II)

wherein $R^1$ is a hydrogen atom or a lower alkyl group and X is a halogen atom; with a secondary amine of the formula (III);

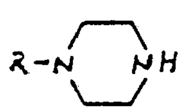

(III)

wherein R is a hydrogen atom or a methyl group; however,

when $R^1$ is a lower alkyl group, the condensed product may be saponified to the carboxylic acid specified above.

3. An antibacterial pharmaceutical composition comprising at least one compound of the formula (I);

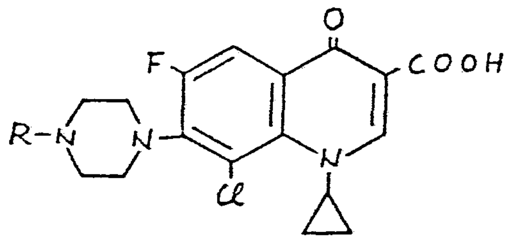

(I)

wherein R is a hydrogen atom or a methyl group; the hydrates or the pharmaceutical acceptable acid addition or alkali salts thereof and an inert pharmaceutically acceptable carrier.

0195841

European Patent Office

# EUROPEAN SEARCH REPORT

Application number

EP 85 10 6626

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | BE-A- 899 399 (KYORIN) * Claims 1,3 * | 1,3 | C 07 D 215/56<br>A 61 K 31/495 |
| Y | GB-A-2 057 440 (KYORIN) * Claims 1,15; examples 5,6 * | 1,3 | |
| E | EP-A-0 167 763 (BAYER) * Claims 1,12; examples 19,21; page 34, lines 1-7 * | 1,3 | |
| | | | TECHNICAL FIELDS SEARCHED (Int. Cl.4) |
| | | | C 07 D 215/00<br>A 61 K 31/00 |

The present search report has been drawn up for all claims

| Place of search | Date of completion of the search | Examiner |
|---|---|---|
| THE HAGUE | 24-06-1986 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document